# EUROPEAN PATENT SPECIFICATION

(11) **EP 3 148 488 B1**
(45) Date of publication and mention of the grant of the patent: **26.02.2025**
(21) Application number: 15798691.0
(22) Date of filing: 29.05.2015
(51) Int. Cl.: A61F 5/56

(54) **ORAL APPLIANCE FOR TREATING SLEEP APNEA**
MUNDVORRICHTUNG ZUR BEHANDLUNG VON SCHLAFAPNOE
APPAREIL BUCCAL POUR LE TRAITEMENT DE L'APNÉE DU SOMMEIL

(30) Priority: 29.05.2014 US 201462004867 P
(43) Date of publication of application: 05.04.2017
(73) Proprietor: R.I.P., LLC, Chatsworth, CA 91311 (US)
(72) Inventor: VEIS, Robin, Chatsworth, CA 91311 (US)
(74) Representative: Gassner, Birgitta
(86) International application number: PCT/US2015/033423
(87) International publication number: WO 2015/184419

(56) References cited:
- WO-A1-02/071989
- WO-A2-2007/002350
- US-A- 5 868 138
- US-A1- 2002 150 856
- US-A1- 2005 199 247
- US-A1- 2009 036 889
- US-A1- 2009 036 889
- US-B2- 7 637 262
- US-B2- 8 127 769

## Description

### BACKGROUND

Sleep apnea is a disorder characterized by abnormal pauses in breathing or instances of abnormally low breathing during sleep. Each pause in breathing, called an apnea, can last from a few seconds to minutes (typically lasting 20 to 40 seconds) and may occur 5 to 30 times or more an hour. Sleep apnea results from a partial-to-complete blockage of a subject's airway. Increased air speed through the airway causes an increase in dynamic pressure and a corresponding drop in static pressure. The decreased static pressure can in some instances draw back the lower jaw and tongue and thereby block the airway. This blockage can increase to the point of becoming complete, which at least temporarily interrupts breathing.

In other instances, interruptions in breathing during sleep may be caused by the collapse of part of a subject's airway where the tongue and upper throat meet the soft palate and uvula. During waking hours, normal muscle tone in most individuals maintains the tissues in these areas in adequate spatial relationships so that air can flow freely, but during sleep some muscle tone is lost, and this loss of muscle tone can result in the partial blockage of the passages at the back of the mouth. Such blockage is generally referred to as obstructive sleep apnea.

A number of oral appliances have been designed to address the problem of sleep apnea. The documents US2005/199247A, US2009/036889A, and WO02/071989 are related prior art documents. US2005/199247A discloses a dental appliance for the treatment of sleep disorders. The appliance includes fasteners extending buccally from the outer surface of the upper tray. US2009/036889A (which is the document closest to the present invention) discloses an apparatus for treatment of sleep apnea employing bone screws implanted into a patient's anterior maxillary bone. WO02/071989 describes a devices for use in treating sleep apnea comprising clips for securing the device to a dentition of the user.

However, there remains a need for an improved oral appliance to treat individuals with sleep apnea.

### FIGURES

Figure 1 is a left front perspective view of one embodiment of the present oral appliance.

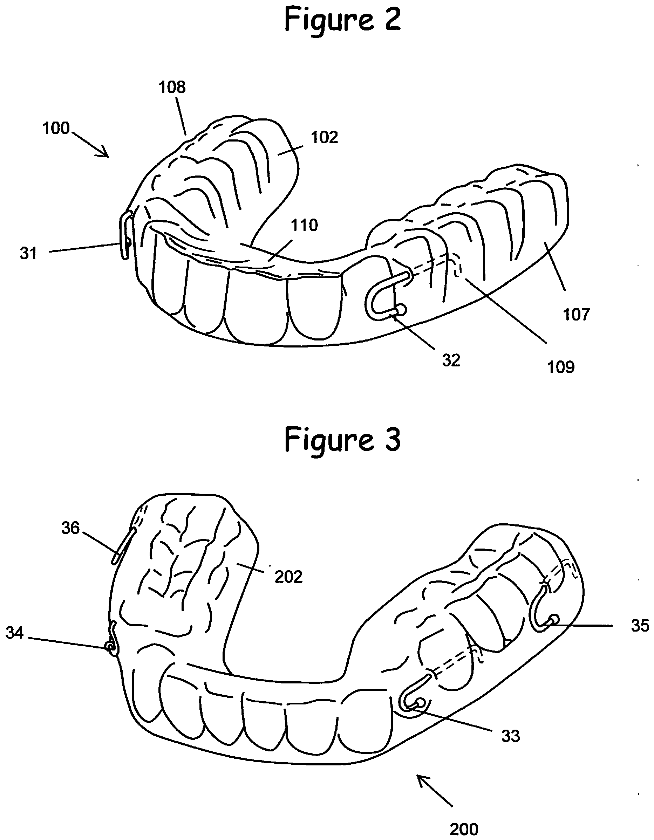

Figure 2 is a left front perspective view of the lower surface of the upper portion of the appliance of Figure 1.
Figure 3 is a left front perspective view of the upper surface of the lower portion of the appliance of Figure 1.

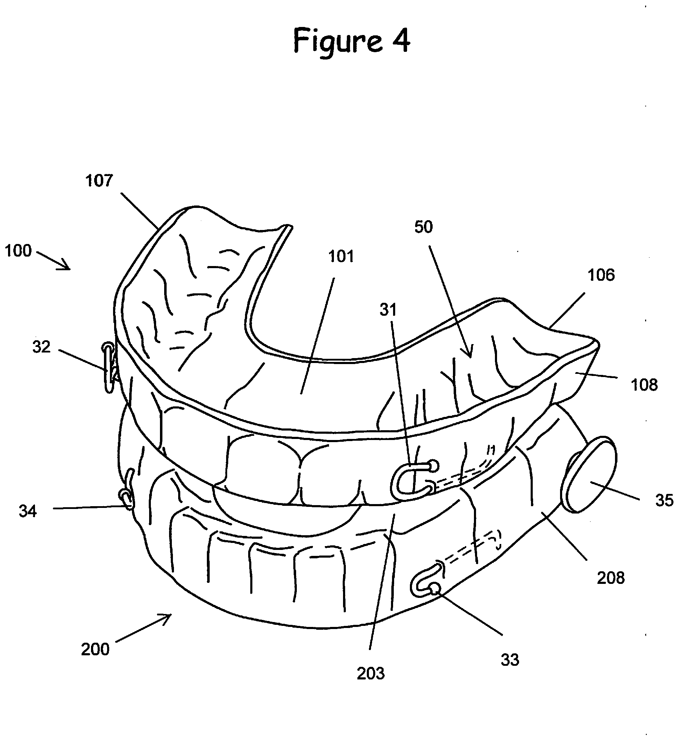

Figure 4 is a left front perspective view of another embodiment of the present oral appliance.

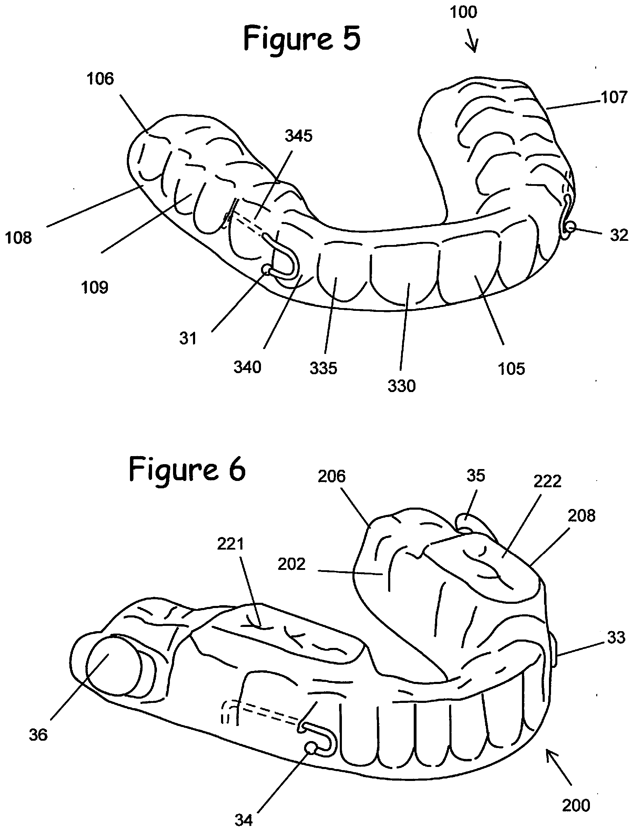

Figure 5 is a right front perspective view of the lower surface of the upper portion of the appliance of Figure 4.
Figure 6 is a right front perspective view of the upper surface of the lower portion of the appliance of Figure 4.

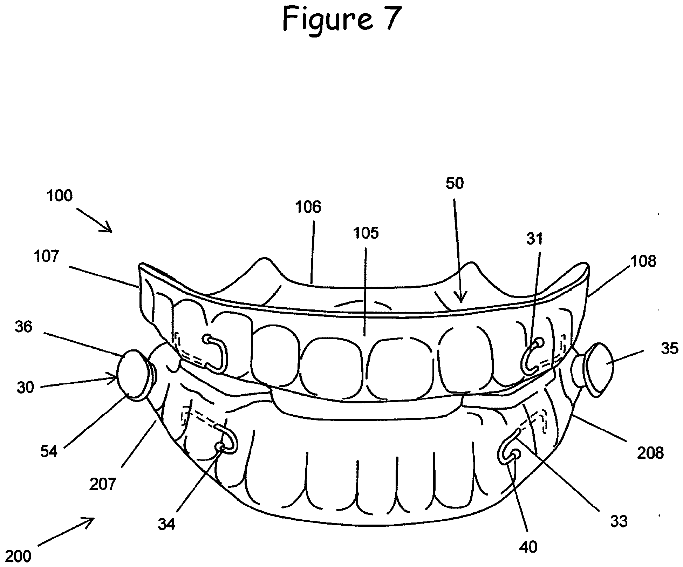

Figure 7 is a front elevation view of the clients of Figure 4.

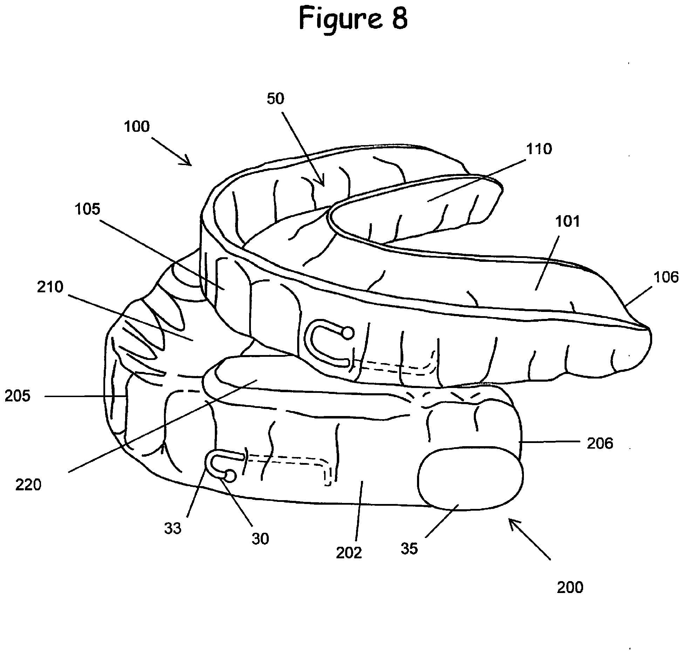

Figure 8 is a side elevation view of the appliance of Figure 4.
Figure 9 is a top plan view of the lower portion of the appliance of Figure 4, additionally including an elastic band extending across the rear of the appliance portion.
Figure 10 is a left front perspective view of the lower portion of the appliance shown in Figure 9.
Figure 11 is a side elevation view of the appliance of Figure 4, additionally including an elastic band extending across the rear of the lower portion of the appliance.

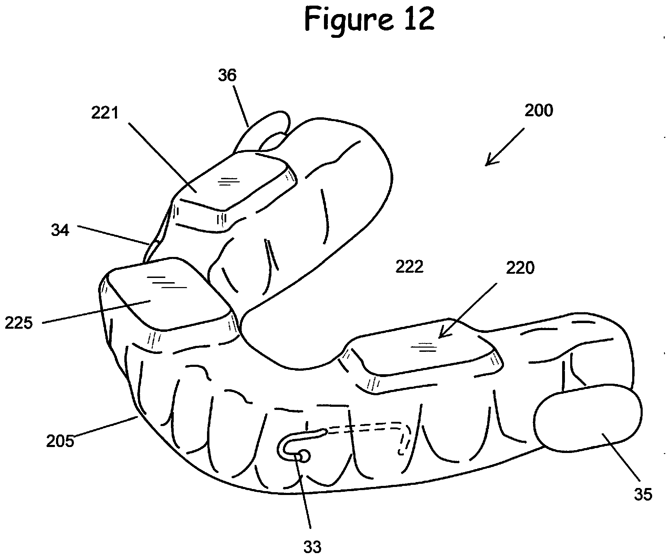

Figure 12 is a left front perspective view of an alternative embodiment of the lower portion of the present appliance.
Figure 13 is a left front perspective view of the lower portion of the appliance of Figure 12, additionally including an elastic band extending across the rear of the appliance portion.

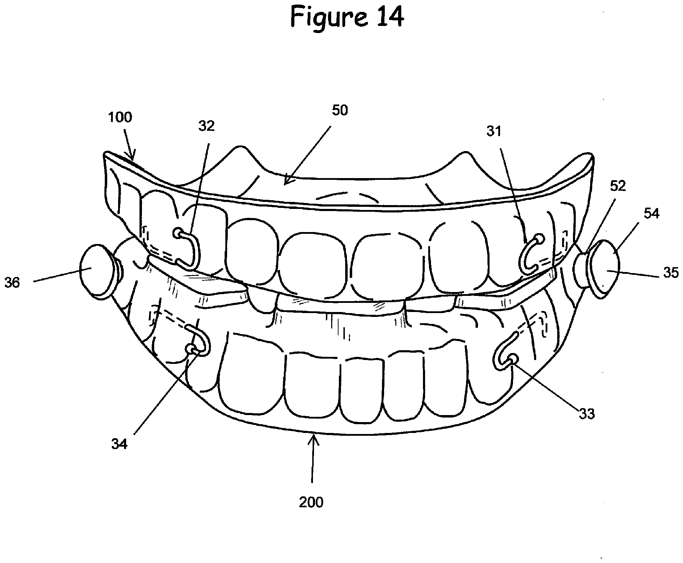

Figure 14 is a front elevation view of an embodiment of the present appliance, including of the lower portion of the appliance shown in Figure 12.
Figure 15 is a left front perspective view of a further embodiment of the present oral appliance.
Figure 16 is a right front perspective view of the lower surface of the upper portion of the appliance of Figure 14.
Figure 17 is a left front perspective view of the upper surface of the lower portion of the appliance of Figure 14.

### SUMMARY

The present invention is an oral appliance for treating snoring and/or sleep apnea. The aspects of the invention are as recited in the appended set of claims. Irrespective of any statement made in the remainder of the description, the embodiments of the invention are solely as detailed in the appended set of claims.

In a preferred embodiment, one elastic band connects the right side fastener of the upper tray to the right side anterior fastener of the lower tray, and a separate elastic band connects the right side fastener of the upper tray to the right side posterior fastener of the lower tray. Likewise, it is preferred that one elastic band connects the left side fastener of the upper tray to the left side anterior fastener of the lower tray, and a separate elastic band connects the left side fastener of the upper tray to the left side posterior fastener of the lower tray. The elastic bands in one embodiment exert a force of no more than 3 Newtons when placed under tension, and preferably exert a force of between 0.9 Newton and 3 Newtons, more preferably exerting a force of between 1.4 Newtons and 2.4 Newtons. The width of the elastic bands is preferably between 4.8 mm and 7.9 mm.

Preferably, the left side anterior fastener of the upper tray is positioned laterally of the portion of the receptacle retaining the left maxillary first bicuspid and the right side anterior fastener of the upper tray is positioned laterally of the portion of the receptacle retaining the right maxillary first bicuspid. Likewise, the left side anterior fastener of the lower tray is positioned laterally of the portion of the receptacle retaining the left maxillary first bicuspid and the right side anterior fastener of the lower tray is positioned laterally of the portion of the receptacle retaining the right maxillary first bicuspid.

The fasteners are preferably buttons or hooks. In some embodiments, all of the fasteners used on an appliance are of the same type, such as hooks, but a mixture of different fasteners can be used in the same appliance. Buttons comprise a stem portion attached at one end to the outer surface of the upper tray or lower tray and have a circumferential portion attached at the other end. In one embodiment, the right side posterior fastener and left side posterior fastener are both buttons. When hooks are used, they are preferably formed from wire, with one end of the wire being secured to the upper tray or lower tray (as the case may be) and the other end extending buccally from the outer surface of the upper tray or lower tray.

In one embodiment, the upper surface of the right and left sides of the lower tray comprise bite surfaces. The bite surfaces are horizontal and are disposed vertically higher than other portions of the upper surface of the lower tray, so that the lower surface of the upper tray contacts the bite surfaces when the upper tray is placed in contact with the lower tray. In a preferred embodiment, the lower tray further comprises an anterior bite surface which is horizontal also disposed vertically higher than other portions of the upper surface of the lower tray such that the lower surface of the upper tray contacts the anterior bite surface when the upper tray is placed in contact with the lower tray.

In another embodiment, the upper tray can include a pair of rear fasteners extending rearwardly from the posterior of the right side and left sides of the tray. The rear fasteners can each include a wire attached to the upper tray at one end and to a button fastener at the other end. An elastic band can be attached to each of these buttons, so that it extends between the right side rear fastener and the left side rear fastener. In a further embodiment, the lower anterior surface of the upper tray can include a central opening to guide placement of the subject's tongue.

In use, prior to sleeping a subject places the present appliance in the subject's mouth, such that the maxillary and mandibular dentition is fitted into the upper and lower appliance portion, respectively. The subject continues wearing the appliance during sleep in order to prevent and/or ameliorate snoring and/or sleep apnea by the subject. The subject can then remove the appliance upon waking.

### Definitions

As used herein, the following terms and variations thereof have the meanings given below, unless a different meaning is clearly intended by the context in which such term is used.

"Anterior" means in the direction of or toward or adjacent the front portion of a subject's mouth and/or in the direction of or toward a source of air pressure.

"Apnea" and "sleep apnea" refer to a temporary cessation of breathing and/or to instances of shallow or infrequent breathing during sleep, generally caused by a blockage of a subject's airway (referred to as obstructive sleep apnea).

"Buccal" means in the direction of or toward a subject's cheek. In relation to a subject's teeth, this refers to the side of the teeth facing the cheek.

"Coronal plane" refers to a hypothetical planar surface that extends through the body from the head to the feet, and divides the body into front and rear halves.

"Coronal surface" refers to the biting surface of a tooth. In posterior teeth this surface is generally referred to as an occlusal surface, while on anterior teeth the term incisal surface can be used.

"Downward" and "downwardly" mean in the direction of or toward a lower portion of a subject's body. "Upward" and "upwardly" mean in the opposite direction, i.e. in the direction of or toward an upper portion of a subject's body.

"Elongated" refers to a configuration or shape having a length which is longer than its width.

"Fastener" refers to a component of the present appliance that mechanically joins or affixes two or more objects together. Fasteners are used to retain elastic bands in the present appliance, and can take the form of a button or hook.

"Horizontal," with respect to the present appliance, refers to disposition in a plane approximately perpendicular to the sagittal and/or the coronal plane of a subject, i.e. within 15 degrees of a perpendicular plane.

"Lateral" means away from the sagittal plane of a subject.

"Labial" means in the direction of, toward, or adjacent to a subject's lips. In relation to a subject's teeth, this refers to the side of the front teeth facing the lips.

"Left" means to the left of the sagittal plane of a subject, from the perspective of the subject.

"Lingual" means in the direction of, toward, or adjacent to a subject's tongue. In relation to a subject's teeth, this refers to the side of the teeth facing the tongue.

"Lower" refers to the relative position of a component in the present appliance which is closer to or toward a lower portion of a subject's body when being used. "Upper" and "higher" refer to the relative position of a component in the present appliance which is closer to or toward an upper portion of a subject's body when being used.

"Mandibular dentition" refers to the teeth of the lower jaw.

"Maxillary dentition" refers to the teeth of the upper jaw.

"Mechanically connected" means physically connected, either through a connection based on direct physical contact or via another mechanical structure.

"Medial" means toward the sagittal plane of a subject.

"Posterior" and "rearward" means in the direction of or toward or adjacent the rear portion of a subject's mouth.

"Right" means to the right of the sagittal plane of a subject, from the perspective of the subject.

"Sagittal plane" refers to an imaginary plane that travels vertically from the top to the bottom of the body of a subject, dividing it into left and right portions.

"Subject" refers to a user of the present appliance, usually a human user.

"Thermoplastic" refers to a material, generally a polymer material, which may be softened by heat and hardened by cooling in a reversible physical process. The thermoplastic materials used in some components of the present appliance retain their shape at 37.8 degrees Celcius (100 degrees Fahrenheit) and preferably become soft (deformable) at a temperature of 100 degrees Celcius (212 degrees Fahrenheit) or below.

"Tray" and "dental tray," as used herein, refer to a portion of the present appliance comprising an open area for receiving the teeth of a subject, preferably including a rim on the lingual portion of the tray and/or on the facial portion of the tray.

"Treat" and "treatment" refer to an intervention which attenuates, prevents, or cures a physiological or medical condition of a subject.

"Vertical," with respect to the present appliance, refers to disposition in a plane approximately parallel to the sagittal and/or the coronal plane of a subject, i.e. within 15 degrees of such a parallel plane. Preferably, vertical refers to a direction toward or away from a subject's head or feet.

The term "comprise" and variations of the term, such as "comprising" and "comprises," are not intended to exclude other additives, components, integers or steps. The terms "a," "an," and "the" and similar referents used herein are to be construed to cover both the singular and the plural unless their usage in context indicates otherwise.

### Oral Appliance

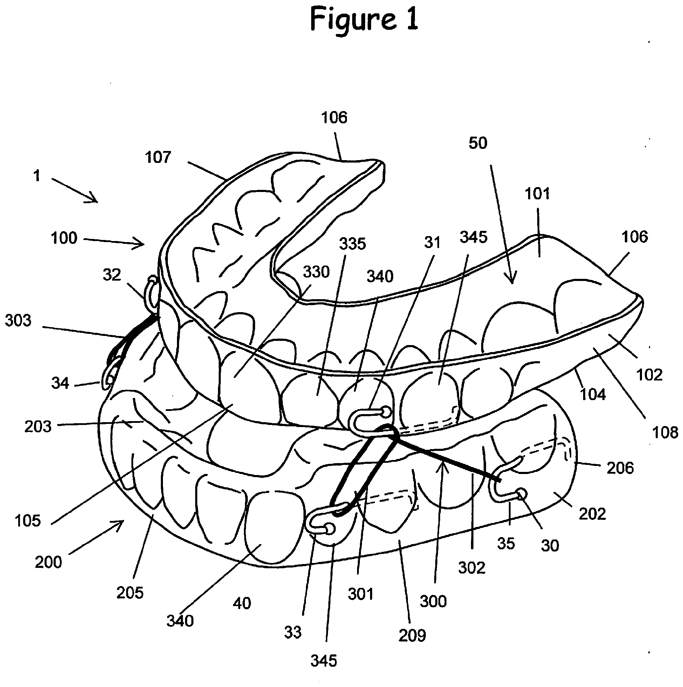

Figure 1 illustrates one embodiment of the present oral appliance. The present appliance 1 generally includes an upper tray 100, a lower tray 200, and elastic bands 300. The upper tray 100 has an inner surface 101, an outer surface 102, an upper surface 103, a lower surface 104, an anterior portion 105, a posterior portion 106, a right side 107, a left side 108, a buccal surface 109, and a labial surface 110. The lower tray 200 has an inner surface 201, an outer surface 202, an upper surface 203, a lower surface 204, an anterior portion 205, a posterior portion 206, a right side 207, a left side 208, a buccal surface 209, and a labial surface 210.

As can be seen in Figure 1, the upper tray 100 is forming a receptacle 50 which is formed to retain the maxillary teeth of a user of the present appliance 1. In the illustrated embodiment, the receptacle is formed so that the inner surface 101 matches the maxillary dentition of the user, in order to provide a comfortable and tights fit. The receptacle can contain, for example, one or more inner surfaces 101 corresponding to the exterior surfaces of the central incisors 330, lateral incisors 335, cuspids 340, first bicuspids 345, second bicuspids 350, and/or molars 355 of a user. In some embodiments, it is not necessary receptacle 50 to contain matching services for all of user's teeth, as long as the upper tray 100 can be securely retained on the user's maxillary teeth during use. Such as embodiments can make use of additional securing mechanisms, such as ball clasps and other mechanisms known to the art. The lower tray 200 is formed with a receptacle for retaining the mandibular teeth of the user in the same manner as the upper tray 100.

In order to assemble the present appliance 1, the upper tray 100 is placed over the lower tray 200, such that the lower surface 104 contacts the upper surface 203 of the lower tray 200. In use, this will generally be done by placing the user's maxillary teeth in the upper tray 100 and placing the user's mandibular teeth in the lower tray 200 and then contacting trays. Elastic bands 300 are then used to connect each of the fasteners 30 on the lower tray 200 to a fastener 30 on the upper tray 100. The elastic bands 300 extend between fasteners 30 located on the outer, buccal surfaces of the trays. An upper right side fastener 32 extends buccally from the outer surface 102 of the upper tray 100 adjacent the portion of the receptacle 50 retaining the right maxillary first bicuspid 345, between the cuspids 340 and second bicuspids 350. The upper right side fastener 32 (and likewise the upper left side fastener 31, anterior lower right side fastener 34, and anterior lower left side fastener 33) can in some embodiments overlap the portion of a receptacle 50 that retains the cuspids 340 and/or second bicuspids 350 of a user. This fastener 30 is connected to a right side lower anterior fastener 34 extending buccally from the outer surface 202 of the lower tray 202 adjacent the portion of the receptacle retaining the right mandibular first bicuspid 345 and/or the right mandibular second bicuspid 350 by attaching an elastic band 300 (elastic band 301) to both the upper right side fastener 32 and the right side lower anterior fastener 34. Preferably, the elastic bands 300 are in the form of circular bands, as are commonly used in orthodontic appliances, and elastic bands 301 is a fixed by stretching it from the upper right side fastener 32 to the right side lower anterior fastener 34.

The upper right side fastener 32 is likewise connected to a posterior lower right side fastener 36 located on the buccal surface 209 in the posterior portion 205 of the lower tray 200, preferably adjacent the portion of the receptacle 50 of the lower tray 200 retaining one of the posterior molars 355, such as the second molar or third molar. The upper right side fastener 32 is connected to the posterior lower right side fastener 36 with an elastic band 302, in the same manner that the elastic band 301 connects upper right side fastener 32 to right side lower anterior fastener 34. Preferably, separate elastic bands 300 are used to connect the upper right side fastener 32 to each of the right side lower anterior fastener 34 and the posterior lower right side fastener 36, but in some embodiments a single elastic band 300 may be used. The upper left side fastener 31 is connected by elastic bands 303 and 304 to a left side lower anterior fastener 33 and the posterior lower left side fastener 35 in the same manner as previously described for the right side of the present appliance 1.

The fasteners 30 can be any of a number of fasteners known to the art. In the illustrated embodiments, hooks 40 and buttons 30 are exemplified. The hooks 40 comprise a proximal end 42 which is attached to the buccal surface 109, 209 of a respective tray of the present appliance 1, for example by being embedded within a polymer comprising the upper tray 100 or lower tray 200, respectively. The distal ends 44 of the hooks extend outwardly, and preferably extend anteriorly so as not to extend unduly toward a subject's cheek. Hooks 40 typically include a bend, in order to help retain the elastics 300.

The buttons 30 used in the present appliance can include a laterally (horizontally) extending portion 52 to which an elastic band 300 can be secured. A circumferential "button" portion 54 extending away from the axis of the laterally extending portion 52 is preferably included at the distal end of a buccal button 30 in order to better secure an elastic band 300 on the laterally extending portion 52. The circumferential portions 54 are preferably ovoid or elliptically-shaped and are oriented approximately parallel to the buccal surface of the upper or lower tray.

The present appliance 1 in some embodiments includes a bite plane or surface 220 on the upper surface of the lower tray 200, on both the right side and the left side, forming a right side bite surface 221 and a left side bite surface 222. The bite surface 220 are horizontal and are each disposed vertically higher than other portions of the upper surface of the lower tray so that the lower surface of the upper tray contacts the bite surfaces 220 when the upper tray 100 is placed in contact with the lower tray 200. The flat occlusal bite plane 220 is preferably placed over the bicuspid/first molar area of the lower tray 200, opening the vertical axis of the appliance 1. The anterior area between the upper and lower segments is left open to allow for tongue space and unencumbered breathing. In one embodiment, an anterior bite surface 225 is provided on the upper surface of the anterior portion of the lower tray 200, in order to provide a three-point fit together with the right side bite surface 221 and a left side bite surface 222.

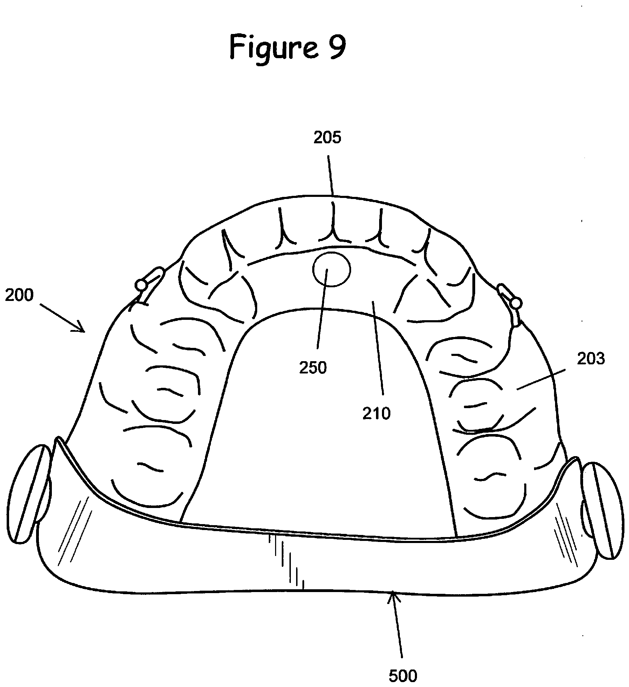

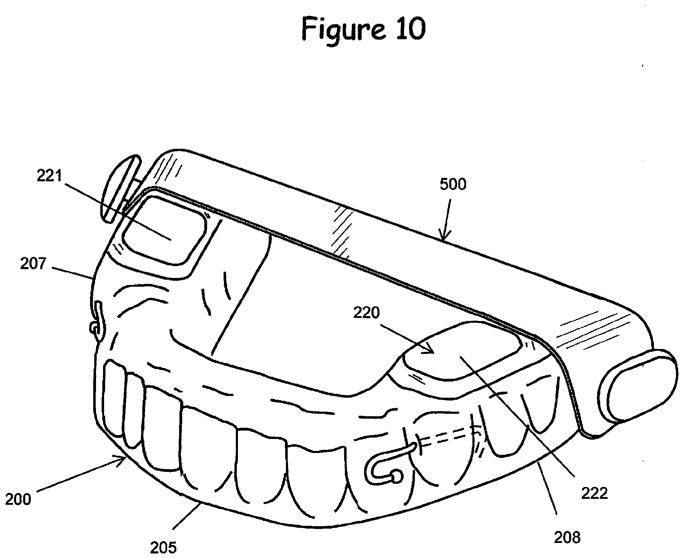

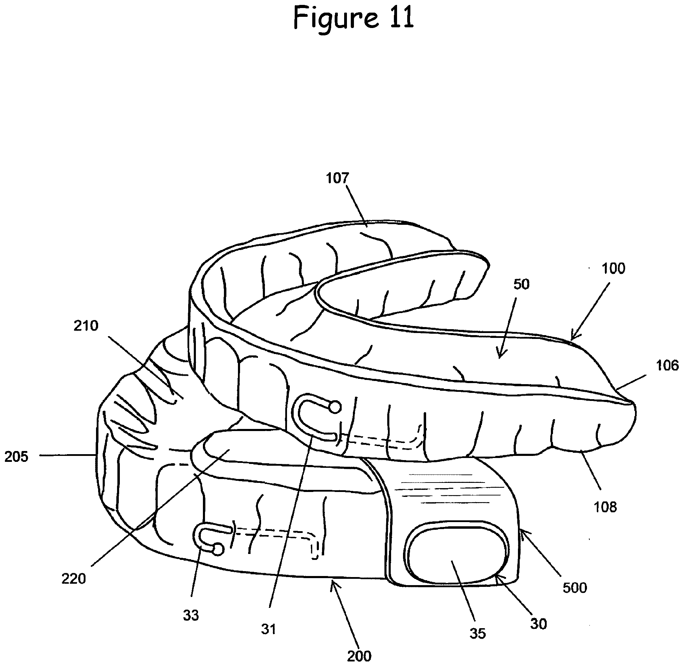

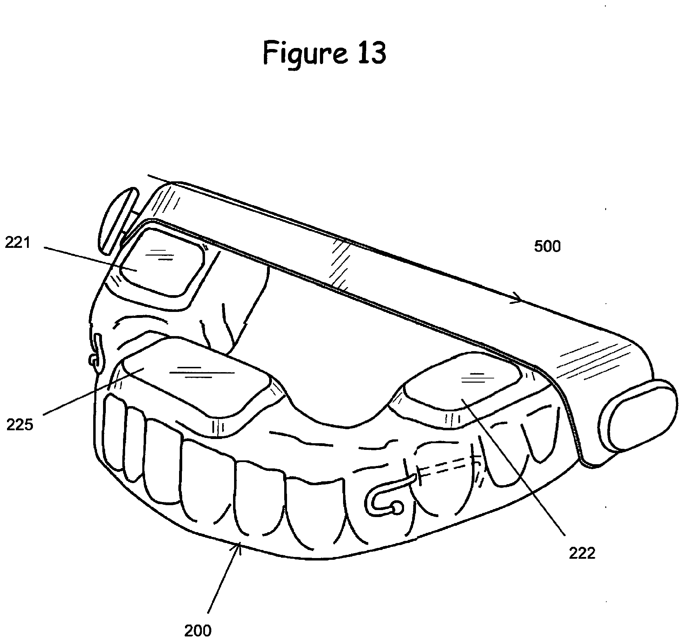

As shown in Figures 9-11 and 13, a laterally extending band 500 can be optionally used in some embodiments of the present appliance, to restrain a user's tongue, and in these embodiments the bite surfaces 220 provide a vertical space in the posterior portion of the present appliance so that the band 500 can be attached at either end to buttons 30.

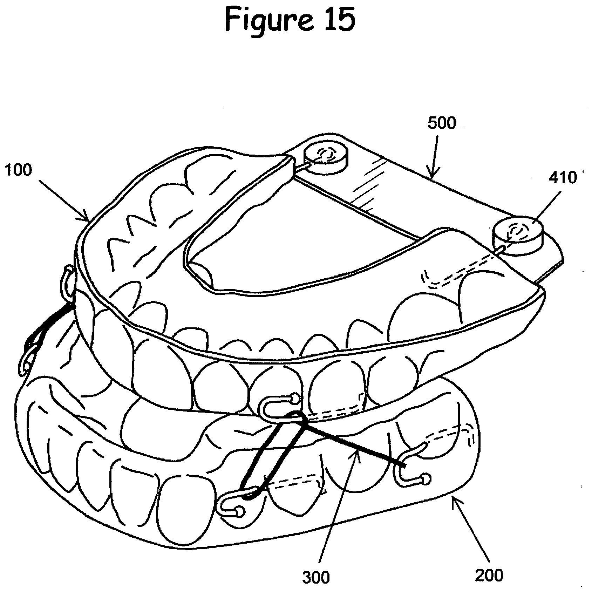

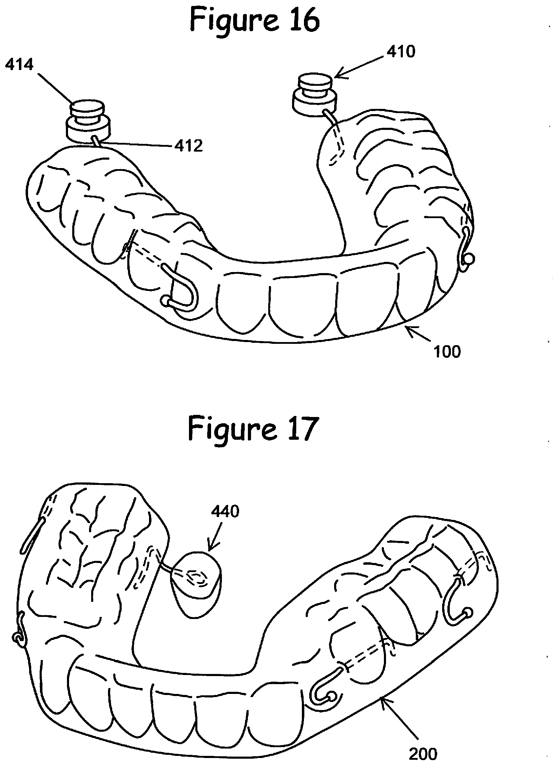

In an alternative embodiment, shown in Figures 15 and 16, the laterally extending band 500 can be attached to the upper tray 100 with retainers 410 in order to restrain a user's tongue. The retainers 410 extend rearwardly from the posterior end of the upper tray. In the illustrated embodiment, a wire 412 is attached at a proximal end to the upper tray 100 (such as by being embedded in the tray material), and at the distal end a fastener 414 is provided to attach to the band 500. In the illustrated embodiment, the fastener 414 comprises a button portion for insertion into a corresponding opening in the band 500. Through the use of wires or other rigid but flexible (adjustable) connectors, the position of the band 500 can be adjusted in order to better treat a user's snoring or apnea, and/or for better comfort.

In a further alternative embodiment, the lower tray 200 can be provided with tongue support pads 440 extending laterally inward from the labial surface of the lower tray 200. Such pads are likewise preferably attached to the lower tray 200 with wires or other rigid but flexible (adjustable) connectors so that their position can be adjusted. As shown in Figure 9, the lower tray 200 can also be provided with an opening 250 in the lower anterior surface of the lower tray to guide placement of the subject's tongue.

One of the advantages of the present appliance 1 is that standard orthodontic elastic bands, such as continuous, generally circular elastic bands 300, can be used to apply a light force to keep the upper tray 100 and lower tray 200 in the proper relative position to one another in a subject's mouth. Elastics connecting the upper fasteners to the lower anterior fasteners control vertical positioning of the appliance while the elastics connecting the upper fasteners to the lower posterior fasteners control anterior-posterior movement of the subject's mandible and jaw. The use of such lower force elastics in this manner provides greater comfort and less pressure on teeth.

The elastic bands used in the present appliance 1 can be 3/16 inch (4.8 mm), ¼ inch (6.35 mm), and/or 5/16 inch (7.87 mm) in diameter, with 3/16 inch being preferred. The amount of force imparted by the bands can be varied by using bands of different weights, for example bands having weights of 170 grams, 227 grams, 454 grams and/or 680 grams (6 ounces, 8 ounces, 16 ounces and/or 24 ounces).

The weight of the bands varies with the thickness of the band material. Although the force imparted by a band can be as much as 4 or 6 Newtons, forces of between 0.9 Newton and 3 Newtons are preferred, more preferably a force of between 1.4 Newtons and 2.4 Newtons. The force exerted by an elastic band can be measured, for example, with a universal testing machine, such as the Emic DL 500MF testing machine (Emic Co, Sao Paulo, Brazil). Non-latex elastic bands are preferably used, in order to avoid an allergic reaction by a subject, and the bands preferably have a diameter of 3/16 inch (5 millimeters).

### Materials

The trays of the present oral appliance can be formed from a variety of orally compatible materials, typically polymers. In one embodiment, acrylic is used to form the present appliance. Thermoplastic polymers are typically used in the present appliance, but thermosets, thermoplastic elastomers, and other materials can also be used. The thermoplastic materials that are used must be capable of retaining their shape when used by a subject, and thus must remain solid at about 37.8 degrees Celcius (100 degrees Fahrenheit) and preferably remain solid at somewhat higher temperatures, such as at 43.3 degrees Celcius (110 degrees Fahrenheit), 48.9 degrees Celcius (120 degrees Fahrenheit) or higher. The materials used to form the present trays also preferably become deformable at a temperature of 100 degrees Celcius (212 degrees Fahrenheit) or less, so that they can be made plastic by being placed in boiling water. Preferably, the material is not deformable at less than 48.9 degrees Celcius (120 degrees Fahrenheit), preferably at not less than 62.8 degrees Celcius (145 degrees Fahrenheit).

In preferred embodiments, the thermoplastic materials are copolymers of ethylene and vinyl acetate. In such embodiments, vinyl acetate is used in an amount of at least 25% by weight, more preferably between 25% and 35% by weight. Commercially available materials of this type are currently used in oral appliances known as "boil and bite" appliances. One such material is an ethylene vinyl acetate copolymer manufactured by the DuPont Company under the trade name ELVAX^{®}.

The use of such thermoplastic materials, commonly used in "boil and bite" dental appliances, allows the present appliance to be produced and fitted to a subject's mouth inexpensively and conveniently. The walls forming the interior passageways of the appliance are designed to allow such a relatively soft and less rigid material to be used to form the present appliance but still allow such interior passageways to be present in the appliance. In alternative embodiments, the present appliance can be formed from more rigid materials, preferably plastic, which can be produced by a laboratory or other specialist for a subject.

### Method of Manufacture (the below method does not fall inside the scope of protection)

The present appliance can be formed in ways known to the art, such as by injection molding. The molded blank can then be imparted with a dental impression of a subject's teeth, so that the appliance can be securely retained in the mouth of the subject. This can be performed using known methods. For example, when the present appliance is a "boil and bite" type appliance, the upper dental tray 100 and lower dental tray 200 are first evaluated for their fit with a subject's mouth. Different sizes of the present appliance can be provided for patients with differently sized mouths. After this, the trays of the appliance can be softened, preferably by placing the appliance in near boiling water for between several seconds and one minute. The softened appliance is then placed in the subject's mouth in alignment with the subject's upper and lower teeth, and the subject is instructed to bite into the softened material to make an impression of the teeth in the softened material. The tray material is then allowed to cool in the mouth for approximately one minute, after which the appliance is preferably soaked in cold water for an additional minute. Creating a customized dental impression in the trays of the present appliance in other ways and using other materials can be accomplished by one of skill in the art using known methods.

### Method of Use (the below method does not fall inside the scope of protection)

Once the trays 100, 200 have been customized for a particular subject, elastic bands 300 are placed on the buccal fasteners as shown in Figure 1. The lateral posterior elastic band (optional) runs across the arch from the midpoint on the posterior molars. The occlusal build-up on the lower segment allows for clearance of the blue cross-arch elastic. The slope in the buccal portion on the lower tray 200 allows for proper placement of the anterior-posterior (diagonal) elastic 320. This elastic runs from the anterior of the upper tray 100 to the posterior of the lower tray 200 as shown. The amount of desired advancement is controlled by selection of elastic size and strength. The elastic that is placed vertically from the buttons in the first bicuspid region on both trays allows for a "closed-mouth" posture during sleep.

Although the present invention has been described in considerable detail with reference to certain preferred embodiments, other embodiments are possible. The steps disclosed for the present methods, for example, are not intended to be limiting nor are they intended to indicate that each step is necessarily essential to the method, but instead are exemplary steps only. Therefore, the scope of the appended claims should not be limited to the description of preferred embodiments contained in this disclosure.

Recitation of value ranges herein is merely intended to serve as a shorthand method for referring individually to each separate value falling within the range. Unless otherwise indicated herein, each individual value is incorporated into the specification as if it were individually recited herein.

## Claims

1. A sleep apnea oral appliance, comprising:
(a) an upper tray (100) having an upper surface (103), a lower surface (104), an anterior portion (105), a posterior portion (106), a right side (107), a left side (108), a buccal surface (109), a labial surface (110), and an outer surface, the upper tray (100) comprising:
an upper tray receptacle (50) formed in the upper surface (103), the upper tray receptacle having an inner surface (101) formed to retain the maxillary teeth of a subject;
a right side upper tray fastener (32) extending buccally from the outer surface of the upper tray (100) adjacent the portion of the upper tray receptacle retaining the right maxillary first bicuspid and/or the right maxillary second bicuspid; and
a left side upper tray fastener (31) extending buccally from the outer surface of the upper tray (100) adjacent the portion of the upper tray receptacle retaining the left maxillary first bicuspid and/or the left maxillary second bicuspid;
(b) a lower tray (200) having an upper surface (203), a lower surface (204), an anterior portion (205), a posterior portion (206), a right side (207), a left side (208), a buccal surface (209), a labial surface (210), and an outer surface, the lower tray (200) comprising:
a lower tray receptacle (50) formed in the lower surface (204), the lower tray receptacle (50) having an inner surface (201) formed to retain the mandibular teeth of the subject;
a right side anterior fastener (34) extending buccally from the outer surface of the lower tray (200) adjacent the portion of the lower tray receptacle (50) retaining the right mandibular first bicuspid and/or the right mandibular second bicuspid;
a left side anterior fastener (33) extending buccally from the outer surface of the lower tray (200) adjacent the portion of the lower tray receptacle (50) retaining the left mandibular first bicuspid and/or the left mandibular second bicuspid;
a right side posterior fastener (36) extending buccally from the outer surface of the lower tray (200) adjacent the portion of the lower tray receptacle (50) retaining a right mandibular molar; and
a left side posterior fastener (35) extending buccally from the outer surface of the lower tray (200) adjacent the portion of the lower tray receptacle (50) retaining a left mandibular molar; and
(c) a plurality of orthodontic elastic bands (300) including:
a first orthodontic elastic band (301) connecting the left side upper tray fastener (31) of the upper tray (100) to the left side anterior fastener (33),
a second orthodontic elastic band (302) connecting the left side upper tray fastener (31) of the upper tray (100) to the left side posterior fastener (35) of the lower tray (200),
a third orthodontic elastic band (303) connecting the right side upper tray fastener (32) of the upper tray (100) to the right side anterior fastener (34) of the lower tray (200), and
a fourth orthodontic elastic band (304) connecting the right side upper tray fastener (32) of the upper tray (100) to the right side posterior fastener (36) of the lower tray (200),
wherein the orthodontic elastic bands exert a force of between 0.9 Newton and 3 Newtons under tension, and
wherein the lower jaw is free to move forward during use.

2. The appliance of claim 1, wherein the orthodontic elastic bands (300) exert a force of between 1.4 Newtons and 2.4 Newtons when placed under tension.

3. The appliance of claims 1 or 2, wherein the width of the orthodontic elastic bands (300) is between 4.8 mm and 7.9 mm.

4. The appliance of claim 1, wherein the left side anterior fastener (33) of the upper tray (100) is positioned laterally of the portion of the upper tray receptacle retaining the left maxillary first bicuspid and the right side anterior fastener (34) of the upper tray (100) is positioned laterally of the portion of the upper tray receptacle retaining the right maxillary first bicuspid.

5. The appliance of claim 1, wherein the left side anterior fastener (33) of the lower tray (200) is positioned laterally of the portion of the lower tray receptacle (50) retaining the left maxillary first bicuspid and the right side anterior fastener (34) of the lower tray (200) is positioned laterally of the portion of the lower tray receptacle (50) retaining the right maxillary first bicuspid.

6. The appliance of any of claims 1-5, wherein one or more of the fasteners (30) are buttons comprising a stem portion and a circumferential portion, wherein the stem portion is attached at a proximal end to the outer surface of the upper tray (100) or lower tray (200), the stem being attached at a distal end to the circumferential portion.

7. The appliance of any of claims 1-6, wherein one or more of the fasteners (30) are hooks (40).

8. The appliance of any of claims 1-7, wherein the upper surface (203) of the right side (207) of the lower tray (200) comprises a right side bite surface (221) and the upper surface (203) of the left side (208) of the lower tray (200) comprises a left side bite surface (222), wherein the bite surfaces are horizontal and are each disposed vertically higher than other portions of the upper surface of the lower tray (200), such that the lower surface (104) of the upper tray (100) contacts the bite surfaces when the upper tray (100) is placed in contact with the lower tray (200).

9. The appliance of claim 8, wherein the lower tray (200) further comprises an anterior bite surface (225), wherein the anterior bite surface is horizontal and is disposed vertically higher than other portions of the upper surface (203) of the lower tray (200) such that the lower surface (104) of the upper tray (100) contacts the anterior bite surface when the upper tray (100) is placed in contact with the lower tray (200).

## Patentansprüche

1. Orale Schlafapnoe-Vorrichtung, umfassend:
(a) eine obere Schale (100) mit einer oberen Oberfläche (103), einer unteren Oberfläche (104), einem vorderen Abschnitt (105), einem hinteren Abschnitt (106), einer rechten Seite (107), einer linken Seite (108), einer bukkalen Oberfläche (109), einer labialen Oberfläche (110) und einer äußeren Oberfläche, wobei die obere Schale (100) umfasst:
eine Oberschalen-Aufnahme (50), die in der oberen Oberfläche (103) ausgebildet ist, wobei die Oberschalen-Aufnahme eine innere Oberfläche (101) aufweist, die ausgebildet ist, um die Oberkieferzähne eines Subjekts zu halten;
ein rechtsseitiges Unterschalen-Befestigungsmittel (32), das sich bukkal von der äußeren Oberfläche der unteren Schale (100) benachbart zu dem Abschnitt der Oberschalen-Aufnahme erstreckt, welcher den ersten rechten Oberkiefer-Prämolar und/oder den zweiten rechten Oberkiefer-Prämolar hält; und
ein linksseitiges Oberschalen-Befestigungsmittel (31), das sich bukkal von der äußeren Oberfläche der oberen Schale (100) benachbart zu dem Abschnitt der Oberschalen-Aufnahme erstreckt, welcher den ersten linken Oberkiefer-Prämolar und/oder den zweiten linken Oberkiefer-Prämolar hält;
(a) eine untere Schale (200) mit einer oberen Oberfläche (203), einer unteren Oberfläche (204), einem vorderen Abschnitt (205), einem hinteren Abschnitt (206), einer rechten Seite (207), einer linken Seite (208), einer bukkalen Oberfläche (209), einer labialen Oberfläche (210) und einer äußeren Oberfläche, wobei die untere Schale (200) umfasst:
eine Unterschalen-Aufnahme (50), die in der unteren Oberfläche (204) ausgebildet ist, wobei die Unterschalen-Aufnahme (50) eine innere Oberfläche (201) aufweist, die ausgebildet ist, um die Unterkieferzähne des Subjekts zu halten;
ein rechtsseitiges vorderes Befestigungsmittel (34), das sich bukkal von der äußeren Oberfläche der unteren Schale (200) benachbart zu dem Abschnitt der Unterschalen-Aufnahme (50) erstreckt, welcher den ersten rechten Unterkiefer-Prämolar und/oder den zweiten rechten Unterkiefer-Prämolar hält;
ein linksseitiges vorderes Befestigungsmittel (33), das sich bukkal von der äußeren Oberfläche der unteren Schale (200) benachbart zu dem Abschnitt der der Unterschalen-Aufnahme (50) erstreckt, welcher den ersten linken Unterkiefer-Prämolar und/oder den zweiten linken Unterkiefer-Prämolar hält;
ein rechtsseitiges hinteres Befestigungsmittel (36), das sich bukkal von der äußeren Oberfläche der unteren Schale (200) benachbart zu dem Abschnitt der Unterschalen-Aufnahme (50) erstreckt, welcher einen rechten Unterkiefer-Molar hält; und
ein linksseitiges hinteres Befestigungsmittel (35), das sich bukkal von der äußeren Oberfläche der unteren Schale (200) benachbart zu dem Abschnitt der Unterschalen-Aufnahme (50) erstreckt, welcher einen linken Unterkiefer-Molar hält; und
(c) eine Vielzahl von kieferorthopädischen elastischen Bändern (300), die beinhalten:
ein erstes kieferorthopädisches elastisches Band (301), welches das linksseitige Oberschalen-Befestigungsmittel (31) der oberen Schale (100) mit dem linksseitigen vorderen Befestigungsmittel (33) verbindet,
ein zweites kieferorthopädisches elastisches Band (302), welches das linksseitige Oberschalen-Befestigungsmittel (31) der oberen Schale (100) mit dem linksseitigen hinteren Befestigungsmittel (35) der unteren Schale (200) verbindet,
ein drittes kieferorthopädisches elastisches Band (303), welches das rechtsseitige Oberschalen-Befestigungsmittel (32) der oberen Schale (100) mit dem rechtsseitigen vorderen Befestigungsmittel (34) der unteren Schale (200) verbindet; und
ein viertes kieferorthopädisches elastisches Band (304), welches das rechtsseitige Oberschalen-Befestigungsmittel (32) der oberen Schale (100) mit dem rechtsseitigen hinteren Befestigungsmittel (36) der unteren Schale (200) verbindet,
wobei die kieferorthopädischen elastischen Bänder unter Spannung eine Kraft zwischen 0,9 Newton und 3 Newton ausüben, und
wobei das Unterkiefer sich während der Verwendung frei nach vorne bewegen kann.

2. Vorrichtung nach Anspruch 1, wobei die kieferorthopädischen elastischen Bänder (300) eine Kraft zwischen 1,4 Newton und 2,4 Newton ausüben, wenn sie unter Spannung gesetzt werden.

3. Vorrichtung nach Anspruch 1 oder 2, wobei die Breite der kieferorthopädischen elastischen Bänder (300) zwischen 4,8 mm und 7,9 mm beträgt.

4. Vorrichtung nach Anspruch 1, wobei das linksseitige vordere Befestigungsmittel (33) der oberen Schale (100) seitlich des Abschnitts der Oberschalen-Aufnahme positioniert ist, welcher den ersten linken Oberkiefer-Prämolar hält, und das rechtsseitige vordere Befestigungsmittel (34) der oberen Schale (100) seitlich des Abschnitts der Oberschalen-Aufnahme positioniert ist, welcher den ersten rechten Oberkiefer-Prämolar hält.

5. Vorrichtung nach Anspruch 1, wobei das linksseitige vordere Befestigungsmittel (33) der unteren Schale (200) seitlich des Abschnitts der Unterschalen-Aufnahme (50) positioniert ist, welcher den ersten linken Oberkiefer-Prämolar hält, und das rechtsseitige vordere Befestigungsmittel (34) der unteren Schale (200) seitlich des Abschnitts der Oberschalen-Aufnahme (50) positioniert ist, welcher den ersten rechten Oberkiefer-Prämolar hält.

6. Vorrichtung nach einem der Ansprüche 1-5, wobei eines oder mehrere der Befestigungsmittel (30) Knöpfe sind, die einen Schaftabschnitt und einen umlaufenden Abschnitt umfassen, wobei der Schaftabschnitt an einem proximalen Ende an der äußeren Oberfläche der oberen Schale (100) oder der unteren Schale (200) angebracht ist, wobei der Schaft an einem distalen Ende an dem umlaufenden Abschnitt angebracht ist.

7. Vorrichtung nach einem der Ansprüche 1-6, wobei eines oder mehrere der Befestigungsmittel (30) Haken (40) sind.

8. Vorrichtung nach einem der Ansprüche 1-7, wobei die obere Oberfläche (203) der rechten Seite (207) der unteren Schale (200) eine rechtsseitige Bissfläche (221) umfasst und die obere Oberfläche (203) der linken Seite (208) der unteren Schale (200) eine linksseitige Bissfläche (222) aufweist, wobei die Bissflächen horizontal sind und vertikal jeweils höher als andere Abschnitte der oberen Oberfläche der unteren Schale (200) angeordnet sind, so dass die untere Oberfläche (104) der oberen Schale (100) mit den Bissflächen in Kontakt steht, wenn die obere Schale (100) mit der unteren Schale (200) in Kontakt gebracht wird.

9. Vorrichtung nach Anspruch 8, wobei die untere Schale (200) ferner eine vordere Bissfläche (225) umfasst, wobei die vordere Bissfläche horizontal ist und vertikal höher als andere Abschnitte der oberen Oberfläche (203) der unteren Schale (200) angeordnet ist, so dass die untere Oberfläche (104) der oberen Schale (100) mit der vorderen Bissfläche in Kontakt steht, wenn die obere Schale (100) mit der unteren Schale (200) in Kontakt gebracht wird.

## Revendications

1. Appareil buccal pour apnée du sommeil, comprenant :
(a) un plateau supérieur (100) possédant une surface supérieure (103), une surface inférieure (104), une partie antérieure (105), une partie
postérieure (106), un côté droit (107), un côté gauche (108), une surface buccale (109), une surface labiale (110) et une surface externe, le plateau supérieur (100) comprenant :
un réceptacle de plateau supérieur (50) formé dans la surface supérieure (103), le réceptacle de plateau supérieur possédant une surface interne (101) formée de manière à retenir les dents maxillaires d'un sujet ;
un élément de fixation de plateau supérieur côté droit (32) s'étendant buccalement depuis la surface externe du plateau supérieur (100) adjacente à la partie du réceptacle de plateau supérieur retenant la première prémolaire maxillaire droite et/ou la seconde prémolaire maxillaire droite ; et
un élément de fixation de plateau supérieur côté gauche (31) s'étendant buccalement depuis la surface externe du plateau supérieur (100) adjacente à la partie du réceptacle de plateau supérieur retenant la première prémolaire maxillaire gauche et/ou la seconde prémolaire maxillaire gauche ;
(b) un plateau inférieur (200) possédant une surface supérieure (203), une surface inférieure (204), une partie antérieure (205), une partie
postérieure (206), un côté droit (207), un côté gauche (208), une surface buccale (209), une surface labiale (210) et une surface externe, le plateau inférieur (200) comprenant :
un réceptacle de plateau inférieur (50) formé dans la surface inférieure (204), le réceptacle de plateau inférieur (50) possédant une surface interne (201) formée de manière à retenir les dents mandibulaires du sujet ;
un élément de fixation antérieur côté droit (34) s'étendant buccalement depuis la surface externe du plateau inférieur (200) adjacente à la partie du réceptacle de plateau inférieur (50) retenant la première prémolaire mandibulaire droite et/ou la seconde prémolaire mandibulaire droite ;
un élément de fixation antérieur côté gauche (33) s'étendant buccalement depuis la surface externe du plateau inférieur (200) adjacente à la partie du réceptacle de plateau inférieur (50) retenant la première prémolaire mandibulaire gauche et/ou la seconde prémolaire mandibulaire gauche ;
un élément de fixation postérieur côté droit (36) s'étendant buccalement depuis la surface externe du plateau inférieur (200) adjacente à la partie du réceptacle de plateau inférieur (50) retenant une molaire mandibulaire droite ; et
un élément de fixation postérieur côté gauche (35) s'étendant buccalement depuis la surface externe du plateau inférieur (200) adjacente à la partie du réceptacle de plateau inférieur (50) retenant une molaire mandibulaire gauche ; et
(c) une pluralité de bandes élastiques orthodontiques (300) incluant :
une première bande élastique orthodontique (301) reliant l'élément de fixation de plateau supérieur côté gauche (31) du plateau supérieur (100) à l'élément de fixation antérieur côté gauche (33),
une deuxième bande élastique orthodontique (302) reliant l'élément de fixation de plateau supérieur côté gauche (31) du plateau supérieur (100) à l'élément de fixation postérieur côté gauche (35) du plateau inférieur (200),
une troisième bande élastique orthodontique (303) reliant l'élément de fixation de plateau supérieur côté droit (32) du plateau supérieur (100) à l'élément de fixation antérieur côté droit (34) du plateau inférieur (200), et
une quatrième bande élastique orthodontique (304) reliant l'élément de fixation de plateau supérieur côté droit (32) du plateau supérieur (100) à l'élément de fixation postérieur côté droit (36) du plateau inférieur (200),
dans lequel les bandes élastiques orthodontiques exercent une force comprise entre 0,9 Newton et 3 Newtons sous tension, et
dans lequel la mâchoire inférieure est libre de se déplacer vers l'avant lors de l'utilisation.

2. Appareil selon la revendication 1, dans lequel les bandes élastiques orthodontiques (300) exercent une force comprise entre 1,4 Newton et 2,4 Newtons lorsqu'elles sont mises sous tension.

3. Appareil selon la revendication 1 ou 2, dans lequel la largeur des bandes élastiques orthodontiques (300) est comprise entre 4,8 mm et 7,9 mm.

4. Appareil selon la revendication 1, dans lequel l'élément de fixation antérieur côté gauche (33) du plateau supérieur (100) est positionné latéralement par rapport à la partie du réceptacle de plateau supérieur retenant la première prémolaire maxillaire gauche et l'élément de fixation antérieur côté droit (34) du plateau supérieur (100) est positionné latéralement par rapport à la partie du réceptacle de plateau supérieur retenant la première prémolaire maxillaire droite.

5. Appareil selon la revendication 1, dans lequel l'élément de fixation antérieur côté gauche (33) du plateau inférieur (200) est positionné latéralement par rapport à la partie du réceptacle de plateau inférieur (50) retenant la première prémolaire maxillaire gauche et l'élément de fixation antérieur côté droit (34) du plateau inférieur (200) est positionné latéralement par rapport à la partie du réceptacle de plateau inférieur (50) retenant la première prémolaire maxillaire droite.

6. Appareil selon l'une quelconque des revendications 1 à 5, dans lequel un ou plusieurs des éléments de fixation (30) sont des boutons comprenant une partie tige et une partie circonférentielle, dans lequel la partie tige est fixée au niveau d'une extrémité proximale à la surface externe du plateau supérieur (100) ou du plateau inférieur (200), la tige étant fixée au niveau d'une extrémité distale à la partie circonférentielle.

7. Appareil selon l'une quelconque des revendications 1 à 6, dans lequel un ou plusieurs des éléments de fixation (30) sont des crochets (40).

8. Appareil selon l'une quelconque des revendications 1 à 7, dans lequel la surface supérieure (203) du côté droit (207) du plateau inférieur (200) comprend une surface de morsure côté droit (221) et la surface supérieure (203) du côté gauche (208) du plateau inférieur (200) comprend une surface de morsure côté gauche (222), dans lequel les surfaces de morsure sont horizontales et sont chacune disposées verticalement plus haut que les autres parties de la surface supérieure du plateau inférieur (200), de sorte que la surface inférieure (104) du plateau supérieur (100) entre en contact avec les surfaces de morsure lorsque le plateau supérieur (100) est mis en contact avec le plateau inférieur (200).

9. Appareil selon la revendication 8, dans lequel le plateau inférieur (200) comprend en outre une surface de morsure antérieure (225), dans lequel la surface de morsure antérieure est horizontale et est disposée verticalement plus haut que les autres parties de la surface supérieure (203) du plateau inférieur (200) de sorte que la surface inférieure (104) du plateau supérieur (100) entre en contact avec la surface de morsure antérieure lorsque le plateau supérieur (100) est mis en contact avec le plateau inférieur (200).
